# EUROPÄISCHE PATENTANMELDUNG

(11) **EP 4 011 271 A1**
(43) Veröffentlichungstag der Anmeldung: **15.06.2022**
(21) Anmeldenummer: 21210414.5
(22) Anmeldetag: 25.11.2021
(51) Int. Cl.: A61B 1/12, A61B 1/06, A61B 1/05, A61B 1/00, A61B 1/015, A61B 1/018, G02B 23/24

(54) **ENDOSKOP MIT ROTATIONSTROMMEL SOWIE BEDIENVERFAHREN**

(30) Priorität: 09.12.2020 DE 102020132778
(71) Anmelder: Karl Storz SE & Co. KG, 78532 Tuttlingen (DE)
(72) Erfinder: Schwarz, Peter, 78532 Tuttlingen (DE)

(57) **Zusammenfassung**

Endoskop (10) mit einer Rotationstrommel (20) oder einem Rotationsmodul, insbesondere für die Verwendung als ein steriles Einweginstrument, mit einem langerstreckten starren und/oder flexiblen Schaftrohr (12), das an einem distalen Ende (14) vorzugsweise mittels einer Lagergabel (16) die Rotationstrommel (20) um zumindest eine erste Rotationsachse (18) rotierbar lagert, in der ein optisches Abbildungssystem (22) angeordnet ist, wobei das optische Abbildungssystem (22) vorzugsweise einen elektronischen Bildaufnehmer (24) und/oder eine Abbildungsoptik (26) und/oder eine Beleuchtungseinrichtung (28) umfasst, wobei die zumindest eine erste Rotationsachse (18) etwa quer zu einer Längsachse (30) des Schaftrohrs (12) verläuft und wobei das Endoskop zumindest eine Steuerleitung (32) aufweist, um die Rotationstrommel (20) vorzugsweise durch eine Linearbewegung zu rotieren. Dabei ist die zumindest eine Steuerleitung (32) der Rotationstrommel (20) auf einer Außenseite/Außenfläche (36) des Schaftrohres (12), bevorzugt einen Arbeitskanal (34) im Inneren des Schaftrohres (12) und den Innendurchmesser des Schaftrohres (12) nicht einschränkend, verläuft und mit zumindest einem Hebelabstand (a) zu der zumindest einen ersten Rotationsachse (18) an der Rotationstrommel (20) befestigt ist.

## Beschreibung

Die vorliegende Erfindung betrifft ein Endoskop mit einer Rotationstrommel, insbesondere für die Verwendung als ein steriles Einweginstrument, nach dem Oberbegriff des Anspruchs 1 und ein Verfahren zur Bedienung des Endoskops.

Gattungsgemäße Endoskope werden insbesondere für medizinische, bevorzugt chirurgische, Operationen als Beobachtungsinstrument eingesetzt, wobei diese für einen minimalinvasiven Eingriff mit möglichst kleinen Außendimensionen ausgeführt sind. Neben dem Einsatz als Beobachtungsinstrument kann das Endoskop insbesondere für laparoskopische, hysteroskopische oder arthroskopische Operationen auch mit medizinischen Instrumenten und/oder Kanälen zur Fluidleitung ausgestattet sein. Besonders bei einer gleichzeitigen Verwendung eines optischen Abbildungssystems mit einem Arbeitskanal für Instrumente und/oder Kanälen für die Fluidleitung ist zumindest die Funktionsweise des Abbildungssystems oder der Querschnitt des Arbeitskanals durch die begrenzenden und kleinen Außendimensionen eines Schaftrohres des Endoskops eingeschränkt. Zum Beispiel kann bei dieser gleichzeitigen Verwendung entweder ein Abbildungssystem mit geringer Auflösung und/oder beschränktem Blickfeld oder alternativ sehr kleine Instrumente mit eingeschränkten Möglichkeiten für die Fluidleitung ausgeführt werden.

Der Schaft oder üblicherweise ein Schaftrohr des Endoskops kann starr oder flexibel oder abschnittsweise flexibel ausgeführt sein. Im Folgenden wird, wenn nicht genauer beschrieben, unter dem Schaftrohr ein starres und/oder flexibles Schaftrohr verstanden.

Ein Endoskop mit einer kugelförmigen Rotationstrommel als Beobachtungsinstrument mit einem vergrößerten Blickfeld ist aus der EP 1 759 629 A1 der Anmelderin bekannt, das an einem langerstreckten starren Schaftrohr zumindest teilweise in der Rotationstrommel ein optisches Abbildungssystem aufnimmt, wobei die Rotationstrommel um eine erste Schwenkachse verschwenkbar ist, die etwa quer zu einer Längsachse des starren Schaftrohres verläuft und wobei die Rotationstrommel um zumindest eine zweite Schwenkachse verschwenkbar ist, die von der ersten Schwenkachse beabstandet ist und etwa quer zur Längsachse des starren Schaftrohres verläuft. Das Endoskop weist einen Steuermechanismus auf, um die Schwenkbewegung der ersten und zweiten Schwenkachse zu steuern, wobei diese mit einem Getriebe mit Getrieberädern im Inneren des starren Schaftrohres, vorzugsweise mittels eines Riemenantriebs angetrieben wird. Durch die Ausführung des Endoskops mit einer zweifach schwenkbaren Rotationstrommel kann besonders das Blickfeld des Abbildungssystems vergrößert werden, wobei jedoch der Steuermechanismus das gesamte Volumen des starren Schaftrohrs in Anspruch nimmt und kein Arbeitskanal im Inneren des starren Schaftrohrs angeordnet werden kann.

Die WO 2017/040692 A1 zeigt ebenfalls ein Endoskop mit einer kugelförmigen Rotationstrommel an einem langerstreckten starren Schaftrohr, insbesondere für die Verwendung als ein Einweginstrument, wobei die Rotationstrommel in einer Lagergabel an einem distalen Ende des starren Schaftrohres drehbar gelagert ist, und mit einer Steuerleitung im Inneren des starren Schaftrohres rotiert werden kann. Die Rotationstrommel weist im Inneren ein Abbildungssystem auf und ist mit elektronischen Elementen im Inneren des starren Schaftrohres verbunden, wobei gleichzeitig ein Fluid durch das Innere des starren Schaftrohres geleitet werden kann. In dieser Ausführungsform eines Endoskops mit Rotationstrommel wird ein Großteil des starren Schaftohres für die elektronischen Elemente des Abbildungssystems und der Steuerleitung aufgebraucht.

Die EP 3 028 623 A1 der Anmelderin zeigt ein Endoskop indem ein Instrument im Inneren des langerstreckten starren Schaftrohres zusammen mit einem Abbildungssystem geführt werden kann. Dabei wird der Innendurchmesser des starren Schaftrohres fast vollständig von dem Abbildungssystem in Anspruch genommen und durch die hermetische Abdichtung des Abbildungssystems ist das Blickfeld eingeschränkt.

Die US 2015/0359420 A1 zeigt beispielsweise ein Endoskop mit einem flexiblen Schaftrohr und einer Rotationstrommel, die in einem geschlossenen Gehäuse an einem starren distalen Ende des flexiblen Schaftrohrs gelagert ist und mittels eines mechanischen Mechanismus im Inneren des flexiblen Schaftrohres steuerbar ist.

Wie bereits erwähnt, ist der Außendurchmesser von Endoskopen, insbesondere deren Schaftrohr, für einen minimalinvasiven medizinischen Einsatz begrenzt und die im Inneren des Schaftrohres vorgesehenen Kanäle oder Arbeitskanäle für verschiedene Funktionen, wie die Leitung von Fluiden, von Instrumenten oder Werkzeugen und der Elektronik für ein Abbildungssystem am distalen Ende des Schaftrohres, müssen vorzugsweise platzsparend aufgeteilt werden. So wird in dem aufgeführten Stand der Technik auf die Kombination der verschiedenen Funktionen ganz oder teilweise verzichtet oder es werden Einschränkungen in den jeweiligen Funktionen vorgenommen. Insbesondere für die Verwendung von verhältnismäßig großen Arbeitskanälen für die Fluid- und/oder Instrumentenführung ist der Bauraum für die Elektronik eines Abbildungssystems oder eines Sensors eingeschränkt, sodass üblicherweise ein kleiner Sensor mit geringer Auflösung für die Bildgebung verwendet wird.

Besonders für medizinische Anwendungen wird ein steriles und autoklavierbares Abbildungssystem mit elektronischen Anschlüssen gefordert, wobei üblicherweise das Abbildungssystem mit elektronischen Anschlüssen und Steuermechanismus hermetisch abgedichtet ist und deshalb durch ein abgedichtetes Gehäuse nur eingeschränkt mit weiteren Funktionen in einer platzsparenden Weise innerhalb des Schaftrohres kombinierbar ist.

Der vorliegenden Erfindung liegt die Aufgabe zugrunde, ein Endoskop vorzuschlagen, das bei Vermeidung der aus dem Stand der Technik bekannten Probleme die Verwendung eines großen Arbeitskanals in Bezug zu dem Innendurchmesser des Schaftrohres ermöglicht, ohne dabei das Blickfeld oder die Auflösung des Abbildungssystems einzuschränken.

Ferner besteht die Aufgabe darin ein Verfahren zum Bedienen des erfindungsgemäßen Endoskops anzugeben.

Diese Aufgabe wird hinsichtlich des Endoskops mit den Merkmalen des unabhängigen Anspruchs 1 und hinsichtlich des Verfahrens mit den Merkmalen des Anspruchs 13 und/oder 14 gelöst.

Vorteilhafte Ausführungsformen sind Gegenstand der Unteransprüche.

Erfindungsgemäß wird ein Endoskop mit einer Rotationstrommel oder einem Rotationsmodul, insbesondere für die Verwendung als ein steriles Einweginstrument, mit einem langerstreckten starren und/oder flexiblen Schaftrohr vorgeschlagen, das an einem distalen Ende vorzugsweise mittels einer Lagergabel die Rotationstrommel um zumindest eine erste Rotationsachse rotierbar lagert, in der ein optisches Abbildungssystem angeordnet ist, wobei das optische Abbildungssystem vorzugsweise einen elektronischen Bildaufnehmer und/oder eine Abbildungsoptik und/oder eine Beleuchtungseinrichtung umfasst, wobei die erste Rotationsachse etwa quer zur Längsachse des Schaftrohrs verläuft und wobei das Endoskop zumindest eine Steuerleitung aufweist, um die Rotationstrommel vorzugsweise durch eine Linearbewegung zu rotieren. Dabei verläuft die zumindest eine Steuerleitung der Rotationstrommel auf einer Außenseite des Schaftrohres, bevorzugt einen Arbeitskanal im Inneren des Schaftrohres und den Innendurchmesser des Schaftrohres nicht einschränkend, und ist mit einem Hebelabstand zu der zumindest einen ersten Rotationsachse an der Rotationstrommel befestigt.

Unter der Rotationstrommel oder dem Rotationsmodul wird im Rahmen der Erfindung allgemein eine Vorrichtung zur Aufnahme eines Abbildungssystems verstanden, vorzugsweise handelt es sich dabei um eine schwenkbare oder rotierbare Vorrichtung an einem distalen Ende des Schaftrohres und wird deshalb im Rahmen der Erfindung vorzugsweise als Rotationstrommel benannt.

Dabei hat die Erfindung erstaunlicherweise erkannt, dass durch die äußere Anordnung der zumindest einen Steuerleitung der Bauraum im Inneren des Schaftrohres für funktionale Arbeitskanäle erweitert werden kann. Der Arbeitskanal kann dabei modular für unterschiedliche oder kombinierte Funktionen verwendet werden, wie zum Beispiel die Leitung von einem Fluid oder unterschiedlichen Fluiden oder einer Vielzahl an Fluiden, insbesondere in unterschiedliche Flussrichtungen, und/oder Instrumenten. Insbesondere kann dann der gesamte Innendurchmesser des Schaftrohres als Arbeitskanal ausgeführt sein. Des Weiteren kann bei gleicher Arbeitskanalgröße der Außendurchmesser verkleinert werden, was insbesondere bei medizinischen Operationen den Heilungsprozess eines Patienten verkürzt, da keine oder kleinere Wunden entstehen und insgesamt weniger Volumen durch das Endoskop verdrängt wird. Vorteilhafterweise ist es mit dem erfindungsgemäßen Endoskop möglich, verschiedene Funktionen zu kombinieren, ohne dabei eine der Funktionsweisen einzuschränken. So kann ein Abbildungssystem mit hoher Auflösung und einem großen Blickfeld bei gleichzeitig nicht eingeschränkter Arbeitskanalgröße verwendet werden. Besonders durch die Verwendung der Rotationstrommel mit Abbildungssystem kann ein relativ großes Blickfeld durch Schwenken der Rotationstrommel mit einer relativ einfach und robust ausgeführten Steuerungsmechanik ermöglicht werden.

Vorzugsweise ist das Endoskop als Einweginstrument ausgeführt, sodass es vorzugsweise nicht erforderlich ist, die Rotationstrommel mit der Lagerstelle und zumindest ein Teil der Lagergabel hermetisch abzudichten, insbesondere da der Bereich der Lagergabel bedingt desinfizierbar ist. Deshalb kann das Endoskop vorzugsweise auch für industrielle Anwendungen geeignet sein.

Gemäß einer bevorzugten Ausführungsform ist die zumindest eine Steuerleitung bevorzugt auf den äußeren Umfang der Rotationstrommel entlang zumindest einer Aufwickelkurve aufwickelbar. Dadurch kann die Steuermechanik besonders einfach ausgeführt werden und es werden vorteilhafterweise keine störungsanfälligen oder wartungsintensiven Zahnräder oder Übersetzungen und deren Lagerung benötigt.

Weiter bevorzugt ist, dass die zumindest eine Steuerleitung der Rotationstrommel als eine Versorgungsleitung für das Abbildungssystem, vorzugsweise als Flexleiterplatte oder als ein Kabel, für elektronische Schaltkreise im Inneren der Rotationstrommel ausgeführt ist. Dadurch kann neben der elektrischen oder elektronischen Ansteuerung der Rotationstrommel auch die mechanische Steuerung, insbesondere Verschwenkung durch die Steuerleitung realisiert werden.

Die Versorgungsleitung kann elektronische Schaltkreise insbesondere einer elektronisch verstellbaren Abbildungsoptik und/oder eines elektronischen Bildaufnehmers oder Sensors des Abbildungssystems und/oder der Beleuchtungseinrichtung, vorzugsweise mit LEDs, aufweisen. Besonders durch die Verwendung des Endoskops als Einweginstrument muss die Versorgungsleitung nicht autoklavierbar sein und kann so mit einem kleinen Querschnitt ausgeführt werden. Durch die gleichzeitige Verwendung der Versorgungsleitung als Steuerleitung kann besonders vorteilhaft auf eine weitere Steuerleitung für die Rotation der Rotationstrommel verzichtet werden.

Weiterhin bevorzugt ist, dass die zumindest eine Steuerleitung der Rotationstrommel zumindest eine erste Fluidleitung umfasst, die auf die Rotationstrommel aufwickelbar ist und dazu ausgebildet ist einen Operationsbereich mit einem Fluid zu spülen.

Unter dem Fluid kann im Rahmen der Erfindung vorzugsweise eine Flüssigkeit und/oder Gas verstanden werden, wobei für einen medizinischen Einsatz vorzugsweise ein steriles Fluid, wie aufbereiteter Sauerstoff als Luft/Gas und eine physiologische Salzlösung als Flüssigkeit verwendbar ist.

Besonders bevorzugt ist die zumindest eine Steuerleitung in einer Aussparung vorzugsweise mit einem Schutzkragen in der Außenfläche des Schaftrohres geführt, wobei die Aussparung parallel zu der Längsachse des Schaftrohres verläuft. Dadurch ist die zumindest eine Steuerleitung platzsparend und formschlüssig auf der Außenseite des Schaftrohres geführt, um einerseits die zumindest eine Steuerleitung vor mechanischen äußeren Einflüssen zu schützen und um andererseits einem Verletzungsrisiko oder Einklemmen von Gewebe während einer Operation vorzubeugen.

Für die Steuerung der Rotationstrommel sind an der Rotationstrommel vorzugsweise Rückstellmittel angeordnet, bevorzugt eine Drehfeder in der Lagerstelle der Rotationstrommel. Dabei wirkt die Drehfeder einem mechanischen Moment der auf Zug beanspruchten Steuerleitung entgegen, um eine geschwenkte Rotationstrommel in eine Ausgangsposition zu versetzen. Die Drehfeder wirkt also vorzugsweise derart einer Bedienung der Steuerleitung entgegen, dass die Steuerleitung für eine Rotation der Rotationstrommel nicht mit einer Druckkraft beaufschlagt werden muss, um eine flexible Steuerleitung verwenden zu können.

Alternativ oder zusätzlich kann die Rotationstrommel durch eine weitere Steuerleitung auf der gegenüberliegenden Seite zurückführbar sein und/oder mit einer formstabilen Steuerleitung oder einem Art Bowdenzug oder einem Art Steuerstab oder Zahnstange auch mittels einer Druckkraft bedienbar sein.

Weiter alternativ kann die weitere Steuerleitung vorzugsweise als ein Draht ausgebildet sein, wobei ein Rückstellmittel, vorzugsweise eine Feder, insbesondere eine Schraubenfeder, bevorzugt in einem Handgriff zur Rückstellung der Rotationstrommel befestigt sein.

Vorzugsweise kann vorgesehen sein, dass der Arbeitskanal zumindest eine zweite Fluidleitung umfasst, modular bevorzugt eine Luftleitung und/oder bevorzugt eine Flüssigkeitszufuhrleitung und eine Flüssigkeitsabfuhrleitung, aufweist. Insbesondere da das Abbildungssystem mit Versorgungsleitung nicht innerhalb des Schaftrohres angeordnet ist, können insbesondere mehrere Kanäle, wie die Luftleitung zusammen mit der Flüssigkeitszufuhrleitung und der Flüssigkeitsabfuhrleitung innerhalb des Schaftrohres vorgesehen sein. Eine Art Überschaft um das Schaftrohr zur Fluidleitung, der den Außendurchmesser des Schaftrohrs vergrößerst, ist vorteilhafterweise nicht notwendig. Die Funktionalität der zumindest einen zweiten Fluidleitung kann die Optikreinigung und/oder Temperierung/Kühlung der Rotationstrommel und/oder das Spülen und Insufflieren eines Fluids in einen Operationsbereich umfassen. Ein Kühlen der Rotationstrommel ist insbesondere aufgrund einer Wärmeentwicklung im Betrieb der elektronischen Komponenten des Abbildungssystems mit elektronischem Bildaufnehmer und/oder Beleuchtungseinrichtung erforderlich.

Allgemein kann die Fluidleitung für eine Temperierung des Schaftrohres und der Rotationstrommel verwendet werden. So kann es alternativ zu einer Kühlung auch erforderlich sein das Abbildungssystems vor einer Operation auf die zu erwartende Umgebungstemperatur in einem Operationsbereich vorzuheizen. Üblicherweise ist ein Anstieg der Umgebungstemperatur zu erwarten und ein Vorheizen kann ein Beschlagen der Abbildungsoptik des Abbildungssystems vorbeugen.

Vorteilhafterweise wird die Luftleitung in einem trockenen Operationsbereich eingesetzt, um die Rotationstrommel nach einer Optikreinigung zu trocknen und/oder die Rotationstrommel und die elektronischen Komponenten des Abbildungssystems zu kühlen.

In einer trockenen Umgebung des Endoskops weist der Arbeitskanal als zumindest eine zweite Fluidleitung vorzugsweise eine Luftleitung, eine Flüssigkeitszufuhrleitung und eine Flüssigkeitsabfuhrleitung auf. Die Optikreinigung des Endoskops findet in einem trockenen Operationsbereich vorzugsweise in einer Reinigungsstellung statt, in der die Rotationstrommel derart rotiert ist, dass das Blickfeld auf das distale Ende des Schaftrohrs und den Arbeitskanal ausgerichtet ist. In dieser Reinigungsstellung kann das Abbildungssystem mit einer Spülflüssigkeit aus der Flüssigkeitszufuhrleitung gespült werden, wobei mit der Flüssigkeitsabfuhrleitung die Spülflüssigkeit entfernbar ist und so ein Austritt an Spülflüssigkeit in einen trockenen Operationsbereich minimiert werden kann. Vorzugsweise mit der Luftleitung lässt sich die Spülflüssigkeit von dem Abbildungssystem entfernen, um eine Störung des Blickfelds des Abbildungssystems durch Tropfenbildung zu verhindern. Weiter bevorzugt kann die Flüssigkeitszufuhrleitung und vorzugsweise auch die Luftleitung als Spritzdüse ausgebildet sein, um die Reinigungswirkung zu verbessern.

Für einen wässrigen oder nassen Operationsbereich oder Umgebung des Endoskops ist vorzugsweise die Flüssigkeitszufuhrleitung und die Flüssigkeitsabfuhrleitung in dem Arbeitskanal angeordnet, um die Rotationstrommel rückseitig zu umspülen und zu temperieren, sowie den Operationsbereich zu spülen. Für die Spülung des medizinischen Operationsbereichs ist bevorzugt vorgesehen, dass das Schaftrohr an einem distalen Ende umfangsseitig Löcher und/oder eine, vorzugsweise schlitzförmige, Öffnung des Arbeitskanals aufweist, um die zumindest eine zweite Fluidleitung, bevorzugt die Flüssigkeitsabfuhrleitung, mit dem Operationsbereich zu verbinden und um ein Spülfluid aus dem Operationsbereich entfernen zu können. Insbesondere ist das Schaftrohr hinter der Rotationstrommel geöffnet, um einen freien Austritt des Spülfluids, insbesondere des Spülwassers, zu gewährleisten.

Vorzugsweise ist der Arbeitskanal zur Aufnahme zumindest eines Instruments ausgebildet und/oder weist zumindest ein Instrument auf, um in dem Operationsbereich und insbesondere in dem Blickfeld des Abbildungssystems bevorzugt medizinische Operationen durchführen zu können. Dabei weist das zumindest eine Instrument an einem distalen Ende ein steuerbares Werkzeug, zum Beispiel ein scherenartiges Schneidewerkzeug oder Stanzwerkzeug, auf. Die Aufnahme des zumindest einen Instruments kann zusätzlich oder alternativ zu der zumindest einen zweiten Fluidleitung vorgesehen sein. Durch die außenliegende Steuerleitung und insbesondere die außenliegende Versorgung des Abbildungssystems kann der relativ große Arbeitskanal für zumindest ein Instrument und gleichzeitig eine Vielzahl an zweiten Fluidleitungen verwendbar sein.

In einer Weiterbildung, ist das Schaftrohr an einem oberen Abschnitt geöffnet, wobei die zumindest eine erste Steuerleitung an einem gegenüberliegenden unteren Abschnitt geführt ist, um vorzugsweise ein flexibles Instrument in eine Richtung senkrecht zu der Längsachse des Schaftrohrs und in den geöffneten oberen Abschnitt zu verstellen/biegen. Dadurch kann das Instrument, insbesondere die funktionellen Teile und Werkzeuge an dem distalen Ende des zumindest einen Instruments, in den Operationsbereich gebracht werden und gleichzeitig kann der Operationsbereich mit dem Abbildungssystem innerhalb der Rotationstrommel verfolgt werden.

Besonders für die Verwendung des Endoskops mit dem zumindest einen Instrument ist vorzugsweise vorgesehen, dass die Lagergabel schwenkbar um eine zweite Rotationsachse an dem Schaftrohr befestigt ist und der Arbeitskanal in einem geschwenkten Zustand der Lagergabel entlang der Längsachse geöffnet ist, um insbesondere das zumindest eine Instrument aus dem Arbeitskanal in den Operationsbereich zu führen, wobei die Lagergabel vorzugsweise mit einer zweiten Steuerleitung steuerbar ist. Die zweite Steuerleitung ist vorzugsweise ebenfalls auf der Außenseite des Schaftrohres geführt, um den Arbeitskanal im Inneren des Schaftrohres nicht einzuschränken. Mit dieser Ausführungsform ist insbesondere ein starres Instrument parallel zu der Längsachse des Schaftrohrs in den Operationsbereich führbar. Des Weiteren ist mittels einer Rotation der Rotationstrommel um die zweite Rotationsachse ein vorzugsweise flexibles Instrument in eine Richtung senkrecht zu der Längsachse des Schaftrohrs biegbar. Neben der Führung des zumindest einen Instruments ermöglicht die schwenkbare Lagergabel vorteilhafterweise ein erweitertes Blickfeld des Abbildungssystems, auch zum Beispiel hinter Hindernisse, Ecken oder einen Blick entlang einer Außenseite des Schaftrohrs. So ist zum Beispiel auch die Funktion der Führung der zumindest einen Steuerleitung entlang des Schaftrohres mittels des Abbildungssystems überprüfbar. Weiterhin kann mit dem geöffneten Arbeitskanal ein Spülen oder Absaugen von einem Fluid aus dem Operationsbereich begünstigt werden.

Vorzugsweise beträgt der Außendurchmesser des Schaftrohres 3mm bis 6mm, wobei bevorzugt die Rotationstrommel diesen Außendurchmesser nicht übersteigt. Solche Schaftrohre sind für eine Vielzahl nicht invasiver medizinischer Operationen geeignet.

In einer weiter bevorzugten Ausführungsform sind mehrere Steuerleitungen auf unterschiedlichen Aufwickelkurven entlang des Umfangs der Rotationstrommel aufwickelbar, insbesondere mit einem unterschiedlichen Abstand zu der Rotationsachse, um bei gleichem Verstellweg der Steuerleitung eine unterschiedliche Drehverstellung der Rotationstrommel auszuführen. Insbesondere der Verstellweg parallel zu der Längsachse des Schaftrohres und die unterschiedliche Drehverstellung kann zu vordefinierten Blickrichtungen des Abbildungssystems oder zu unterschiedlichen Rotationsgeschwindigkeiten führen, wobei bei gleichem Verstellweg der Steuerleitung die Drehverstellung der Rotationstrommel mit kleiner werdendem Abstand zu der ersten Drehachse der Rotationstrommel abnimmt. Auch die für die Drehverstellung notwendige Zugkraft kann durch die unterschiedlichen Abstände eingestellt werden.

Besonders bevorzugt sind die unterschiedlichen Aufwickelkurven stufenweise entlang der ersten Rotationsachse auf dem Umfang der Rotationstrommel angeordnet, wobei vorzugsweise die Aufwickelkurven mit kleiner werdendem Abstand zu der ersten Rotationsachse in Richtung der äußeren Seiten der Rotationstrommel angeordnet sind.

Die Erfindung betrifft auch ein Verfahren zum Bedienen eines, insbesondere vorangehend beschriebenen, Endoskops, um eine Rotationstrommel mit einem Abbildungssystem, vorzugsweise mit einem elektronischen Bildaufnehmer und/oder einer Abbildungsoptik und/oder einer Beleuchtungseinrichtung, zu steuern, wobei zumindest eine Steuerleitung auf einer Außenseite/Außenfläche eines Schaftrohres des Endoskops und einen Arbeitskanal im Inneren des Schaftrohres nicht einschränkend angeordnet und mit zumindest einem Hebelabstand zu einer zumindest einen ersten Rotationsachse an der Rotationstrommel befestigt wird und wobei anschließend an der zumindest einen Steuerleitung gezogen oder gedrückt wird, um die Rotationstrommel zu rotieren.

In einer Weiterbildung wird das Verfahren dazu verwendet das Endoskop, bevorzugt ein Blickfeld des Abbildungssystems, zu reinigen, wobei die Rotationstrommel in einem ersten Schritt von einer Beobachtungsstellung eines Operationsbereichs in eine Reinigungsstellung, in der das Blickfeld auf das distale Ende des Schaftrohrs und den Arbeitskanal ausgerichtet ist, rotiert wird und wobei das im folgende beschriebene Reinigungsverfahren insbesondere für Endoskope in einer trockenen Umgebung oder einem trockenen Operationsbereich geeignet ist, da ein Austritt an Spülflüssigkeit in einen Operationsbereich minimiert werden kann. In einem zweiten Schritt wird/werden das Abbildungssystem und/oder die Abbildungsoptik und/oder die Beleuchtungseinrichtung, insbesondere in dem Blickfeld, mit einem vorzugsweise wässrigen Spülfluid aus der zumindest einen zweiten Fluidleitung, vorzugsweise einer Flüssigkeitszufuhrleitung mit einer Spritzdüse, gespült. Vorzugsweise kann das wässrige Spülfluid mit einer weiteren zweiten Fluidleitung, vorzugsweise eine Flüssigkeitsabfuhrleitung, abgesaugt werden, um den Austritt an Spülflüssigkeit in einen Operationsbereich zu minimieren. Dabei kann die Rotationstrommel geschwenkt werden, um das gesamte Blickfeld zu reinigen. In einem bevorzugten dritten Schritt wird das vorzugsweise wässrige Spülfluid mit einer Fluidströmung, bevorzugt einer Luftströmung, aus einer Luftleitung oder einer weiteren zweiten Fluidleitung entfernt und so das Blickfeld des Abbildungssystems getrocknet. Dieser Schritt ist insbesondere notwendig, falls das Endoskop in einem trockenen Operationsbereich eingesetzt wird. In einem vierten Schritt wird die Rotationstrommel zurück in die Beobachtungsstellung, vorzugsweise die Beobachtungsstellung aus dem ersten Schritt, rotiert.

In einer weiteren Ausführungsform des Verfahrens wird ein Endoskop mit einer schwenkbaren Lagergabel bedient, wobei in einem ersten Schritt das Schaftrohr, zumindest das distale Ende des Schaftrohres, in einen Operationsbereich geführt und in einem zweiten Schritt die Lagergabel um eine zweite Rotationsachse geschwenkt wird, bis zumindest die Rotationstrommel den Arbeitskanal entlang einer Längsachse des Schaftrohres freigibt. In einem dritten Schritt wird ein zumindest ein Instrument aus dem Arbeitskanal herausgeführt, insbesondere in den medizinischen Operationsbereich hinein. Anschließend und in einem bevorzugten vierten Schritt wird die Rotationstrommel mittels der Steuerleitung um eine erste Rotationsachse geschwenkt, um das zumindest eine Instrument mit einem Blickfeld des Abbildungssystems im Operationsbereich zu verfolgen und für eine Bedienperson abzubilden. In einem fünften Schritt kann die Lagergabel um eine zweite Rotationsachse geschwenkt werden, um das zumindest eine Instrument zu bewegen oder zu verbiegen und in einen definierten Operationsbereich auszurichten. Weitere Vorteile und Einzelheiten der Erfindung ergeben sich aus der nachfolgenden Beschreibung bevorzugter Ausführungsformen der Erfindung sowie anhand von lediglich schematischen Zeichnungen.

Es zeigen:
- Fig. 1a:: eine perspektivische Ansicht auf eine Oberseite eines distalen Endes eines Endoskops mit Rotationstrommel und Steuerleitung,
- Fig. 1b:: eine perspektivische Ansicht auf eine Unterseite des Endoskops gemäß der Fig. 1a,
- Fig. 2a:: eine Seitenansicht des Endoskops gemäß der Fig. 1a,
- Fig. 2b:: einen Längsschnitt des Endoskops gemäß der Fig. 2a in einer Ebene senkrecht zu der ersten Rotationsachse der Rotationstrommel,
- Fig. 2c:: einen Längsschnitt des Endoskops gemäß der Fig. 2b mit einer Flüssigkeitsleitung als Steuerleitung der Rotationstrommel,
- Fig. 2d:: eine Draufsicht auf das distale Ende des Endoskops entlang der Längsrichtung des Endoskops,
- Fig. 3a:: einen Längsschnitt des Endoskops gemäß der Fig. 2b mit einer Rotationstrommel in einer Reinigungsstellung,
- Fig. 3b:: einen Querschnitt des Schaftrohres gemäß der Fig. 3a senkrecht zu der Längsachse des Schaftrohres,
- Fig. 4a:: einen Längsschnitt des Endoskops gemäß der Fig. 2b mit einem Loch zur Flüssigkeitsabfuhr,
- Fig. 4b:: eine perspektivische Ansicht auf ein Endoskop gemäß der Fig. 1a mit Löchern zur Flüssigkeitsabfuhr,
- Fig. 4c:: einen Querschnitt des Schaftrohres gemäß der Fig. 3b mit Löchern gemäß der Fig. 4a,
- Fig. 5a:: einen Längsschnitt des Endoskops gemäß der Fig. 2b mit drehbar gelagerter Lagergabel und einem Instrument,
- Fig. 5b:: einen Längsschnitt des Endoskops gemäß der Fig. 5a mit dem Instrument in einem verbogenen Zustand,
- Fig. 6:: eine Seitenansicht eines Endoskops mit schwenkbarer Lagergabel gemäß der Fig. 5a mit einem flexiblen Schaftrohr,
- Fig. 7a:: eine perspektivische Ansicht auf das Endoskop gemäß der Fig. 1a mit Handgriff,
- Fig. 7b:: eine perspektivische Ansicht auf das Endoskop gemäß der Fig. 1a.

Gleiche Elemente beziehungsweise Elemente mit gleicher Funktion sind in den Figuren mit den gleichen Bezugsziffern versehen.

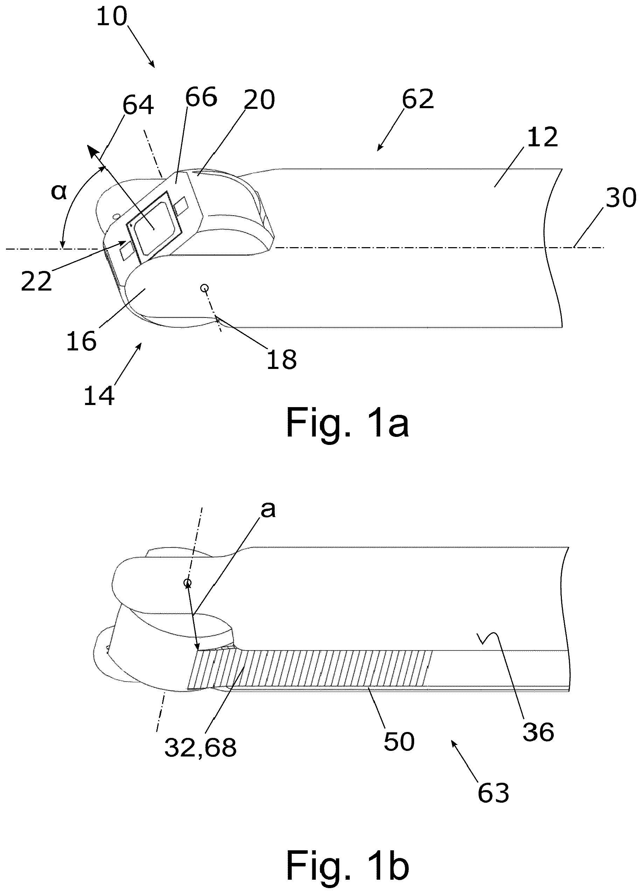

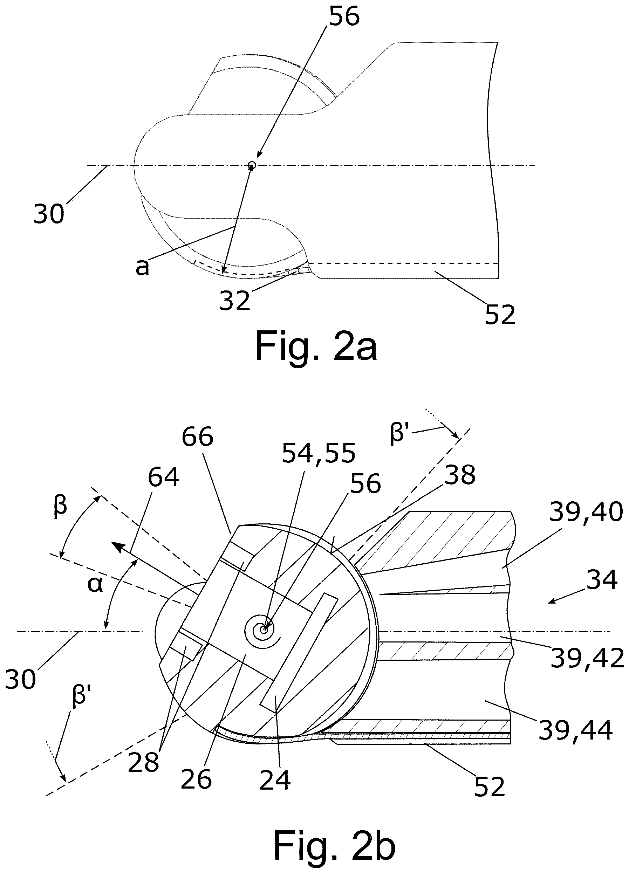

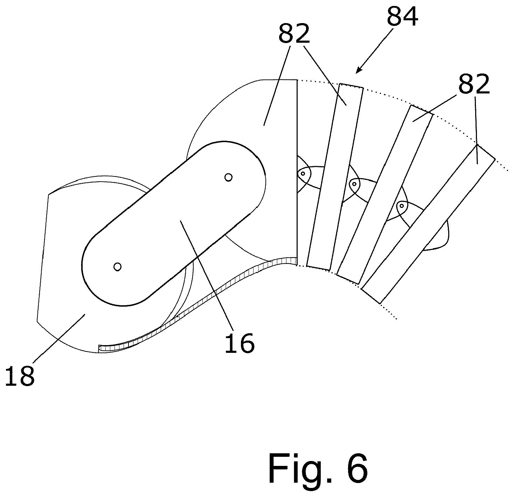

In der Fig. 1a ist ein Endoskop 10 mit einer Rotationstrommel 20 an einem distalen Ende 14 eines langerstreckten starren Schaftrohr 12 dargestellt, wobei die Rotationstrommel 20 mittels einer Lagergabel 16 an dem distalen Ende 14 des Schaftrohrs 12 um eine erste Rotationsachse 18 rotierbar gelagert ist. In der Rotationstrommel 20 ist ein optisches Abbildungssystem 22 angeordnet, welches vorzugsweise einen elektronischen Bildaufnehmer 24, eine Abbildungsoptik 26 und eine Beleuchtungseinrichtung 28, wie in der Fig. 2b im Detail gezeigt, umfasst. Die Rotationstrommel 20 ist von einer Oberseite 62 des Schaftrohrs 12 dargestellt, wobei eine Blickrichtung 64 des Abbildungssystems 22 mit einem Winkel α zu der Längsachse 30 schwenkbar ist und die Lagergabel 16 die Blickrichtung 64 der Abbildungsoptik 26 nicht einschränkt. Durch Schwenken der Rotationstrommel 20 und dadurch der Blickrichtung 64 kann ein Winkel α von bevorzugt über 130° und ein Beobachtungsbereich β`, gemäß der Fig. 2b, abgedeckt werden. Durch Rotation des Schaftrohres 12 um die Längsachse 30 kann der Beobachtungsbereich β` erweitert werden. Mit einem flexiblen distalen Ende 14 des Schaftrohrs 12, gemäß der Fig. 6, ist der gesamte Operationsbereich um das Endoskop 10 herum beobachtbar.

Die erste Rotationsachse 18 verläuft etwa quer zu einer Längsachse 30 des Schaftrohrs 12, wobei das Endoskop 10, wie in der Fig. 1b dargestellt, zumindest eine, vorzugsweise eine Steuerleitung 32 auf einer Unterseite 63 des Schaftrohres 12 aufweist. Diese zumindest eine Steuerleitung 32 ist auf einer Außenseite 36 des Schaftrohrs 12 geführt und mit einem Hebelabstand a zu der ersten Rotationsachse 18 an der Rotationtrommel 20 befestigt. Bevorzugt durch eine Linearbewegung der zumindest einen Steuerleitung 32 parallel zu der Längsachse 30 des Schaftrohrs 12 ist die Rotationstrommel 20 rotierbar.

Alternativ oder abschnittsweise kann das Schaftrohr 12, gemäß der Fig. 6, vorzugsweise an dem distalen Ende 14, auch flexibel mit einer Vielzahl an Lenkungsgliedern 82 ausgestaltet und mittels weiterer nicht dargestellten Steuerleitungen an einem äußeren Rand 84 der Lenkungsglieder 82 schwenkbar sein.

Wie in der Fig. 2b gezeigt, ist ein Arbeitskanal 34 im Inneren des Schaftrohrs 12 und vorzugsweise der Innendurchmesser des Schaftrohres 12 durch die zumindest eine Steuerleitung 32 nicht eingeschränkt.

Die Rotationstrommel 20 ist vorzugsweise kugel- oder zylinderförmig ausgeführt, wobei die Rotationstrommel 20 bevorzugt senkrecht zu einer Blickrichtung 64 des Abbildungssystems 22 abgeflacht ist, um in einem abgeflachten Bereich 66 die Abbildungsoptik 26 und die Beleuchtungseinrichtung 28, vorzugsweise als zwei LEDs, aufzunehmen.

Die zumindest eine Steuerleitung 32 ist vorzugsweise auf zumindest eine Aufwickelkurve 38 entlang des äußeren Umfangs der Rotationstrommel 20 aufwickelbar. Besonders bevorzugt ist die zumindest eine Steuerleitung 32 derart aufwickelbar, dass die Rotationstrommel 20 von einer Blickrichtung 64 entlang der Längsachse 30 des Schaftrohres 12 um zumindest einen Winkel α von 0° bis 180° drehbar ist und so die Blickrichtung 64 in das Innere des Schaftrohres 12 und einen Arbeitskanal 34 ausrichtbar ist. Der Blickwinkel β des Abbildungssystems 22 beträgt vorzugsweise 50° bis 70°, besonders bevorzugt 60°. Dadurch beträgt ein bevorzugter Beobachtungsbereich β', indem das Abbildungssystem mit einem Blickwinkel β einen Operationsbereich durch Schwenken der Rotationstrommel 20 um einen Winkel α aufnehmen kann, zwischen -35° und 215°, besonders bevorzugt zwischen -30° und 210°, wobei der Winkel des Beobachtungsbereich β`, wie auch der Winkel α sich auf eine 0° Richtung entlang der Längsachse 30 des Schaftohres 12 beziehen. Für das Schwenken der Rotationstrommel 20 ist die zumindest eine Steuerleitung 32 zumindest teilweise flexibel ausgeführt, wie in der Fig. 1b mit Querstrichen dargestellt, um diese auf die zumindest eine Aufwickelkurve 38 der Rotationstrommel 20 aufzuwickeln. Dabei kann die zumindest eine Aufwickelkurve 38 entlang des äußeren Umfangs vorzugsweise kreisförmig oder oval ausgeführt sein.

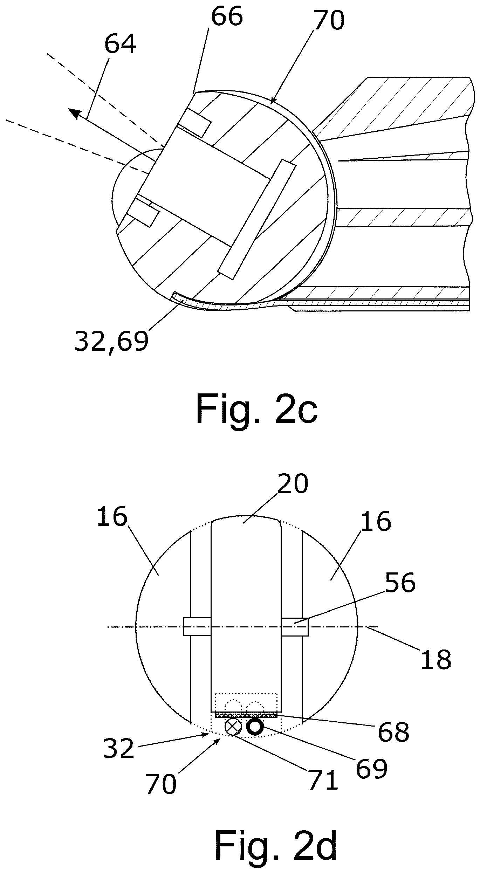

Besonders bevorzugt bildet die zumindest eine Steuerleitung 32 der Rotationstrommel 20 eine Versorgungsleitung für das Abbildungssystem 22 aus, das vorzugsweise als ein in der Fig. 1b dargestellte Flexleiterplatte 68 ausgeführt ist, um elektronische Schaltkreise im Inneren der Rotationstrommel 20, zum Beispiel den elektronischen Bildaufnehmer 24 und/oder eine elektronisch verstellbare Abbildungsoptik 26 und/oder die Beleuchtungseinrichtung 28, zu versorgen. Insbesondere bei der Verwendung der Flexleiterplatte 68 als Steuerleitung 32 ist die entsprechende Aufwickelkurve 38 derart ausgebildet, dass der Biegeradius größer als 0,5mm ist. Alternativ kann das Abbildungssystem 22 auch mit einem Kabel 71, wie in der Fig. 2d dargestellt, elektrisch versorgt sein.

Weiterhin kann vorgesehen sein, dass die zumindest eine Steuerleitung 32, wie in der Fig. 2c und der Fig. 2d dargestellt, zumindest eine erste Fluidleitung 69 umfasst. Dabei ist diese zumindest eine erste Fluidleitung 69 vorzugsweise zusätzlich zu einer elektronischen Versorgungsleitung des Abbildungssystems 22 vorgesehen und auf die Aufwickelkurve 38 aufwickelbar, siehe Fig. 2d.

Die zumindest eine Steuerleitung 32 ist vorzugsweise in einer Vertiefung 70, gemäß der Fig. 2c und der Fig. 2d, auf dem Umfang der Rotationstrommel 20 formschlüssig geführt, damit diese geschützt ist, gleichzeitig der Außendurchmesser der Rotationstrommel 20 nicht weiter vergrößert wird und so auch eine Verletzung von Gewebe in einem Operationsbereich reduziert werden kann.

Wie in der Fig. 1b, der Fig. 2a oder der Fig. 2b dargestellt, ist die zumindest eine Steuerleitung 32 in einer Aussparung 50 auf der Außenseite 36 des Schaftrohrs 12 geführt, wobei die Aussparung 50 bevorzugt parallel zu der Längsachse 30 des Schaftrohrs 12 verläuft und so einen Schutzkragen 52 vorzugsweise im Bereich des distalen Endes 14 des Schaftrohrs 12 ausbildet. Dadurch wird die zumindest eine Steuerleitung 32 geschützt, sowie auch eine Verletzung von Gewebe in einem Operationsbereich durch die bewegbare Steuerleitung 32 verhindert. Vorzugsweise verhindert der Schutzkragen 52, dass sich die zumindest eine Steuerleitung 32 senkrecht zu der Längsachse 30 von der Außenseite 36 des Schaftrohrs 12 lösen kann.

Für die Rückstellung einer rotierten Rotationstrommel 20 ist in dem Bereich der Lagerstelle 56, gemäß der Fig. 2b, vorzugsweise eine Drehfeder 55 als Rückstellungmittel 54 angeordnet, wobei die Drehfeder 55 die Rotationstrommel 20 vorzugsweise in einer Reinigungsposition mit einem Winkel α von etwa 180° zwischen der Blickrichtung 64 und der Längsachse 30 des Schaftrohres 12 hält.

Alternativ ist die Rotationstrommel 20 auch mit einer nicht dargestellten zweiten Steuerleitung auf der Oberseite 62 des Schaftrohres 12 zurückstellbar oder die Steuerleitung 32 ist derart formstabil und in der Aussparung 52 geführt, ähnlich eines Bowdenzugs mit abstützender Hülle, dass die Steuerleitung 32 auch mittels einer Druckkraft verstellbar ist.

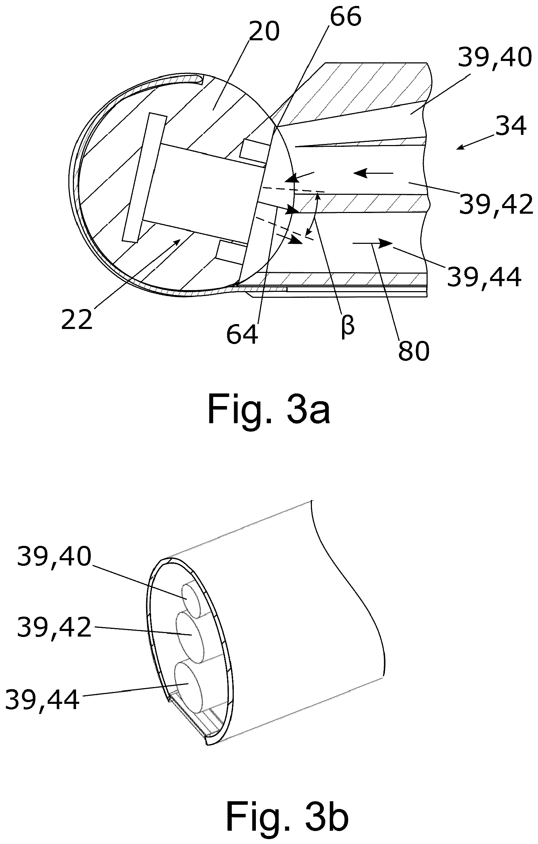

In dem Arbeitskanal 34 des Schaftrohres 12 ist vorzugsweise zumindest eine zweite Fluidleitung 39, bevorzugt eine Luftleitung 39, 40 und/oder bevorzugt eine Flüssigkeitszufuhrleitung 39, 42 und eine Flüssigkeitsabfuhrleitung 39, 44 angeordnet. Die Luftleitung 39, 40 und die Flüssigkeitsleitungen 39, 41, 44 sind in der Fig. 2b und der Fig. 3b dargestellt. In einer Beobachtungsstellung der Rotationstrommel 20, gemäß der Fig. 2b, kann die zumindest eine zweite Fluidleitung 39 zur rückseitigen Kühlung/Temperierung des Abbildungssystems 22 verwendet werden. Insbesondere für die Reinigung des Abbildungssystems 22 von Endoskopen 10 in einem trockenen Operationsbereich, ist die bevorzugt die Rotationstrommel 20 in eine Reinigungsstellung gemäß der Fig. 3a, verdrehbar, wobei die Rotationstrommel 20 mit der Blickrichtung 64 in Richtung des Arbeitskanals 34 geneigt ist. In dieser Reinigungsstellung ist vorzugsweise mit einer Luftleitung 39, 40 in Kombination mit der Flüssigkeitszufuhrleitung 39, 42 eine Verschmutzung von dem abgeflachten Bereich 66 der Rotationstrommel 20 spülbar und damit das Blickfeld β des Abbildungssystems 22 reinigbar. Vorzugsweise ist das Spülfluid eine Spülflüssigkeit aus der Flüssigkeitszufuhrleitung 39, 42 zur Spülung des Abbildungssystems 22, wobei mit der Flüssigkeitsabfuhrleitung 39, 44 die Spülflüssigkeit entfernbar ist und so ein Austritt an Spülflüssigkeit in einen trockenen Operationsbereich minimiert werden kann. Vorzugsweise wird die Luftleitung 39, 40 dafür verwendet, die Spülflüssigkeit von dem Abbildungssystem 22 und dem abgeflachten Bereich 66 zu entfernen, um eine Störung des Blickfelds β des Abbildungssystems 22 durch Tropfenbildung zu verhindern. Weiter bevorzugt kann die Flüssigkeitszufuhrleitung 39, 42 und vorzugsweise auch die Luftleitung 39, 40 als Spritzdüse ausgebildet sein, um die Reinigungswirkung zu verbessern.

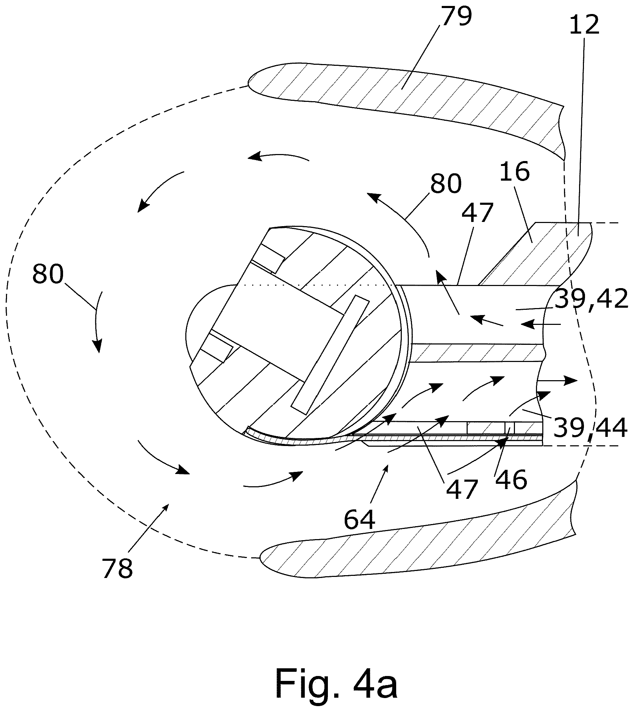

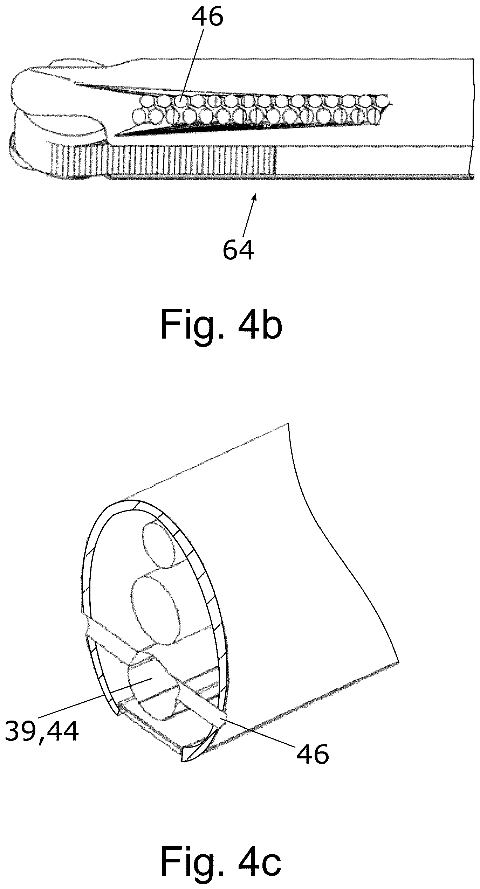

Vorzugsweise für Endoskope 10 mit nasser Umgebungsbedingung oder in einem nassem Operationsbereich kann zusätzlich zu der rückseitigen Kühlung und der Reinigung der Rotationstrommel 20 die zumindest eine zweite Fluidleitung 39 dafür verwendet werden einen Operationsbereich zu spülen, insbesondere um den Operationsbereich in der Blickrichtung 64 des Abbildungssystems 22 in einer Beobachtungsstellung zu spülen. Für eine medizinische Anwendung wird dafür vorzugsweise ein steriles und physiologisches Spülfluid verwendet. Um ein Spülfluid aus dem Operationsbereich zu entfernen wird bevorzugt eine Flüssigkeitsabfuhrleitung 39, 44 mit zumindest einem umfangsseitigen Loch 46 oder einer schlitzförmigen Öffnung 47 an dem distalen Ende 14 des Schaftrohres 12, wie in der Fig. 4a bis Fig. 4c dargestellt, verwendet. Die Löcher 46 und die Öffnung 47 verbinden die Flüssigkeitsabfuhrleitung 39, 44, vorzugsweise auf der Unterseite 64 des Schaftrohres 12, mit dem Operationsbereich. Insbesondere für einen blasenförmigen Operationsbereich 78 mit dem zu operierenden Gewebe 79, gemäß der Fig. 4a, ist die Rotationstrommel 20 in einer Strömungsrichtung 80 umspülbar, wobei die umfangsseitigen Löcher 46 und die Öffnung 47 die Abfuhr des Spülfluids aus dem blasenförmigen Operationsbereich 78 begünstigen. Für diesen Spüleinsatz sind vorzugsweise nur die Flüssigkeitsleitungen 39, 42, 44 erforderlich, da eine Luftleitung 39, 40 in einem nassen Operationsbereich bevorzugt nicht erforderlich ist und das Abbildungssystem 22 gleichzeitig mit dem Spülen des Operationsbereichs reinigbar ist, ohne dabei in eine Reinigungsstellung schwenken zu müssen.

Alternativ oder zusätzlich kann auch der Abstand zwischen der Rotationstrommel 20 und dem Schaftrohr 12 und so die Öffnung 47 vergrößert sein, um einen Zugang zu der Flüssigkeitsabfuhrleitung 39, 44 in der Fig. 4a zu ermöglichen.

Weiterhin ist bevorzugt, dass das Schaftrohr 12, insbesondere die Lagergabel 16, auch an der Oberseite 62 eine Öffnung 47 aufweist, um das Schaftrohr 12 hinter der Rotationstrommel 20 zu öffnen und einen freien Austritt an Spülfluid, insbesondere aus der Flüssigkeitszufuhrleitung 39, 42, zu gewährleisten.

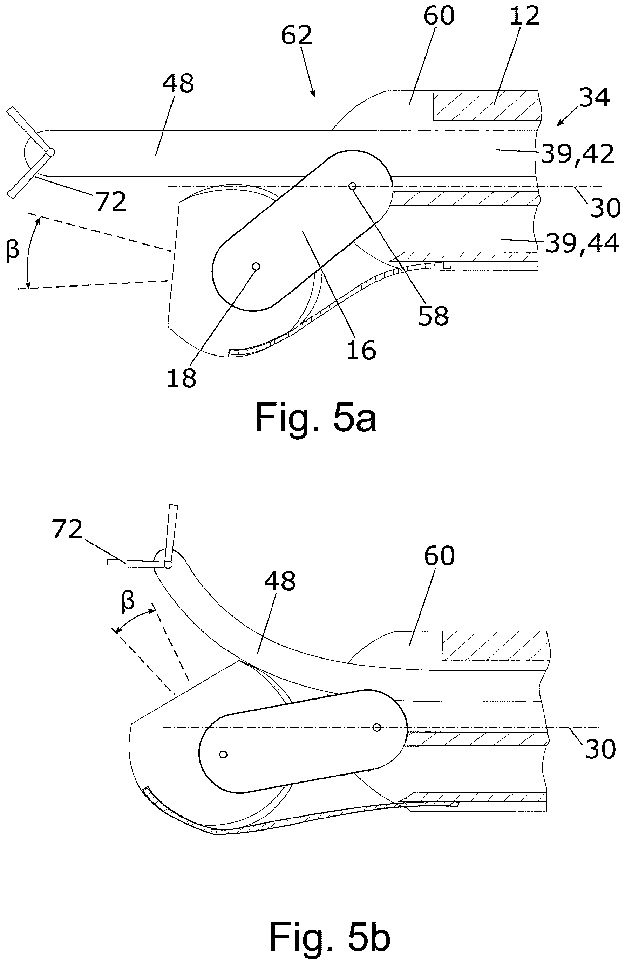

Wie in der Fig. 5a dargestellt, kann der Arbeitskanal 34 alternativ oder zusätzlich zu der zumindest einen zweiten Fluidleitung zur Aufnahme eines Instruments 48 ausgebildet sein und/oder ein Instrument 48 aufweisen. Dieses Instrument 48 kann an einem distalen Ende ein Werkzeug 72, zum Beispiel ein Schneidewerkzeug oder Stanzwerkzeug, aufweisen, das auf die jeweilige medizinische Operation anpassbar ist.

Bevorzugt ist das Schaftrohr 12 auf der Oberseite 62 in einem Abschnitt 60 derart geöffnet, dass ein Instrument 48, vorzugsweise ein flexibles Instrument, in eine Richtung senkrecht zu der Längsachse 30 des Schaftrohres 12 an der Rotationstrommel 20 vorbei verstellbar oder biegbar ist.

Die Fig. 5a zeigt eine weiter bevorzugte Ausführungsform des Endoskops 10 in der die Lagergabel 16 schwenkbar um eine zweite Rotationsachse 58 an dem Schaftrohr 12 befestigt ist und der Arbeitskanal 34 in einem geschwenkten Zustand der Lagergabel 16 entlang der Längsachse 30 derart geöffnet ist, dass vorzugsweise ein starres Instrument 48 aus dem Arbeitskanal 34 in den Operationsbereich führbar ist. Die Lagergabel 16 ist vorzugsweise mit einer nicht dargestellten zweiten Steuerleitung oder einem anderen Bedienmechanismus steuerbar. Vorzugsweise einem Bedienmechanismus für flexible Schaftrohre, der zum Beispiel aus der eingangs genannten US 2015/0359420 A1 bekannt ist. Wie in der Fig. 5b dargestellt, ist mit dieser schwenkbaren Lagergabel 16 insbesondere ein flexibles Instrument 48 mittels der Rotationstrommel 20 in Richtung des oberen geöffneten Abschnitts 60 verstellbar. So kann das Werkzeug 72 am distale Ende des Instruments 48 in einen Operationsbereich bewegt werden, wobei dieses Werkzeug 72 durch eine gleichzeitige Rotation der Rotationstrommel 20 im Blickfeld β des Abbildungssystem 22 bleibt.

Alternativ oder zusätzlich kann die Lagergabel 16 auch mittels eines flexiblen oder elastisch verformbaren Elements, vorzugsweise ein Gummielement, mit dem Schaftrohr 12 verbunden sein, wobei das flexible Element eine passive Rückstellkraft ausbildet, um die Lagergabel 16 um die zweite Rotationsachse 58 zu schwenken und den Arbeitskanal 34 für ein Instrument 48 zu öffnen.

Zusätzlich zur Führung des Instruments 48 kann der Arbeitskanal 34 weitere Kanäle für die Fluidleitung beinhalten, sodass auch bei Verwendung eines Instruments 48 die Rotationstrommel 20 kühlbar oder reinigbar ist und/oder der Operationsbereich spülbar ist. So wirkt vorteilhafterweise die Spülung des Operationsbereiches auch zusätzlich zur Reinigung des Instruments 48. Beispielsweise kann der Arbeitskanal 34 zur Führung des Instruments 48 gleichzeitig ein Flüssigkeitszufuhrkanal 41 sein und das Schaftrohr 12 einen weiteren Flüssigkeitsabfuhrkanal 44 beinhalten.

Wie in der Fig. 6 dargestellt, kann auch ohne ein Instrument die schwenkbare Lagergabel 16 den Biegeradius eines flexiblen Schaftrohres 12 verkleinern oder die Krümmbarkeit vergrößern, oder den von einem starren Endoskop ermöglichen, um zum Beispiel auch ein von dem Schaftrohr 12 selbst abgedecktes Blickfeld des Abbildungssystems 22 zu erfassen oder um hinter Ecken oder Hindernissee blicken zu können. So wird bei einer Rotation der Rotationstrommel 20 und einer gleichzeitigen Rotation der Lagergabel 16 der Beobachtungsbereich auf einen Winkel β' über 180° erweitert, da der Beobachtungsbereich nicht durch das Schaftrohr 12 selbst eingeschränkt ist. Das zumindest abschnittsweise flexible Schaftrohr 12 ist, gemäß der Fig. 6 mit einer Vielzahl an Lenkungsgliedern 82 ausgestaltet und mittels weiterer nicht dargestellter Steuerleitungen an einem äußeren Rand 84 der Lenkungsglieder 82 schwenkbar.

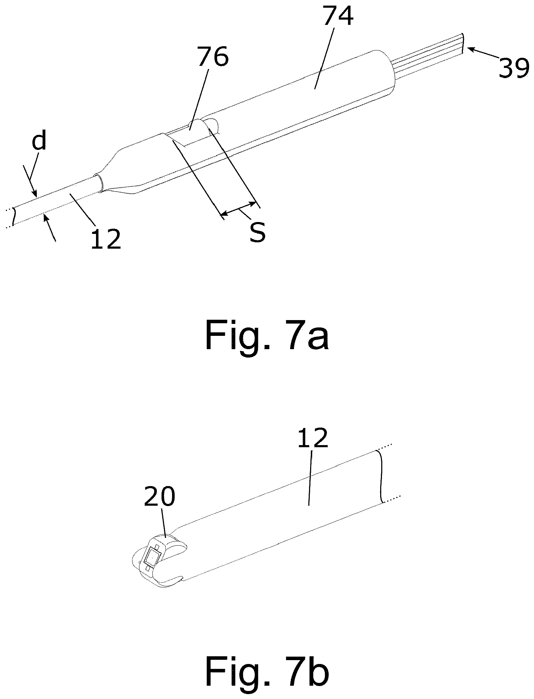

Wie in der Fig. 7a dargestellt, ist die zumindest eine Steuerleitung 32 mittels eines Handgriffs 74 an einem proximalen Ende des Schaftrohrs 12 und einem linear verschiebbaren Bedienelement 76 parallel zu der Längsrichtung 30 des Schaftrohres 12 durch eine Bedienperson verstellbar.

Der Handgriff 74 überführt vorzugsweise auch eine elektronische Kabelführung auf die Flexleiterplatte 68, die vorzugsweise als Steuerleitung 32 linear um einen Verstellweg S verschiebbar ist, um die Rotationstrommel 20 am distalen Ende 14 des Schaftrohrs 12 in der Fig. 7b zu rotieren.

Vorzugsweise ist mittels des Handgriffs 74 auch das Werkzeug 72 des Instruments 48, gemäß der Fig. 5a und der Fig. 5b, bedienbar.

Der Außendurchmesser d des Schaftrohrs 12 beträgt vorzugsweise 3mm bis 6 mm. Solch ein Schaftrohr 12 ist für eine Vielzahl an nicht invasiven medizinischen Operationen an ausgewachsenen Menschen geeignet. Dabei ist bevorzugt die Lagergabel 16 an einem distalen Ende abgerundet und der Durchmesser der Rotationstrommel 20 überschreitet vorzugsweise diesen Außendurchmesser d in eine Richtung senkrecht zu der Längsachse 30 des Schaftrohres 12 nicht, um eine Verletzung in einem medizinischen Einsatz zu minimieren.

Vorzugsweise sind bei der Verwendung von mehreren Steuerleitungen 32 auf unterschiedlichen, hier nicht dargestellten, Aufwickelkurven 38 der Rotationstrommel 20 aufwickelbar, insbesondere mit unterschiedlichen Abständen a zu der Rotationsachse 30, um bei gleichem Verstellweg S der Steuerleitungen 32 entlang der Längsrichtung 30 des Schaftrohres 12 eine unterschiedliche Drehverstellung um den Winkel α auszuführen. Diese unterschiedlichen Aufwickelkurven 38 sind auch bevorzugt, falls ein geringer Abstand a gefordert ist, allerdings die Versorgungsleitung, insbesondere als Flexleiterplatte 68, durch einen maximalen Biegeradius begrenzt ist.

Die Erfindung betrifft auch ein Verfahren zum Bedienen eines vorhergehend beschriebenen Endoskops, wobei die Rotationstrommel 20 von einer Beobachtungsstellung, gemäß der Fig. 2b, in eine Reinigungsstellung, gemäß der Fig. 3a, um einen Winkel α rotiert wird, sodass das Abbildungssystem 22 auf das distale Ende 16 des Schaftrohres 12 und den Arbeitskanal 34 ausgerichtet ist, wobei in dieser Position die zumindest eine zweite Fluidleitung 39 zur Spülung der Abbildungsoptik 26 und/oder der Beleuchtungseinrichtung 28 verwendet wird. Während dem Spülvorgang kann die Rotationstrommel 20 geschwenkt werden, um insbesondere das Blickfeld β des Abbildungssystems 22 zu reinigen. Für Anwendungen in denen kein Spülfluid in den Operationsbereich dringen soll, insbesondere für einen trockenen Operationsbereich, wird vorzugsweise mit einer Flüssigkeitsabfuhrleitung 39, 44 das Spülfluid entfernt. Anschließend an die Spülung mit dem Spülfluid, vorzugsweise einer Spülflüssigkeit, kann die Rotationstrommel 20 vorzugsweise mit einer Luftströmung aus der Luftleitung 39, 40 getrocknet werden, bevor die Rotationstrommel 20 zurück in die Beobachtungsstellung geschwenkt wird, bevorzugt die zuvor verlassene Beobachtungsstellung.

Insbesondere für einen trockenen Operationsbereich ist es oftmals alternativ ausreichend ein durch Feuchtigkeit beschlagene Abbildungsoptik mit einer Luftströmung zu reinigen. Dabei ist der Einsatz einer Spülflüssigkeit vorteilhafterweise nicht erforderlich.

Weiterhin betrifft die Erfindung ein Verfahren um ein Instrument 48 aus dem Schaftrohr 12 zu führen, wobei als erstes das Schaftrohr 12, zumindest das distale Ende 14 des Schaftrohres 12, in einen Operationsbereich geführt wird. Anschließend wird die Rotationstrommel 20 mit der schwenkbaren Lagergabel 16 um eine zweite Rotationsachse 58, gemäß der Fig. 5a, soweit geschwenkt, bis zumindest die Rotationstrommel 20 den Arbeitskanal 34 entlang einer Längsachse 30 des Schaftrohrs 12 freigibt. Danach wird das Instrument 48 aus dem Arbeitskanal 34 herausgeschoben, insbesondere in die Richtung einer Operationsstelle. In einem nächsten Schritt wird die Rotationstrommel 20 mittels der Steuerleitung 32 und um die erste Rotationsachse 18 geschwenkt, um das Instrument 48, insbesondere ein Werkzeug 72 und den Operationsbereich mit dem Blickfeld β des Abbildungssystems 22 zu verfolgen und zu beobachten. Insbesondere für flexible Instrumente kann durch ein Verschwenken der Lagergabel 16 um eine zweite Rotationsachse 58 das Instrument 48, gemäß der Fig. 5b, gebogen werden. In diesem Verfahren kann die Lagergabel 16 aktiv geschwenkt werden oder die Lagergabel 16 kann passiv mittels eines flexiblen oder elastisch verformbaren Elements durch ein Zusammenwirken mit dem Instrument schwenkbar sein.

### Bezugszeichenliste

- 10: Endoskop
- 12: Schaftrohr
- 14: distale Ende des Schaftrohrs
- 16: Lagergabel
- 18: erste Rotationsachse
- 20: Rotationstrommel
- 22: optisches Abbildungssystem
- 24: elektronischer Bildaufnehmer
- 26: Abbildungsoptik
- 28: Beleuchtungseinrichtung
- 30: Längsachse des Schaftrohrs
- 32: zumindest eine Steuerleitung
- 34: Arbeitskanal
- 36: Außenseite des Schaftrohrs
- 38: Aufwickelkurve
- 39: zweite Fluidleitung
- 40: zweite Luftleitung
- 42: Flüssigkeitszufuhrleitung
- 44: Flüssigkeitsabfuhrleitung
- 46: Löcher in dem Schaftrohr
- 47: Öffnung in dem Schaftrohr
- 48: Instrument
- 50: Aussparung
- 52: Schutzkragen
- 54: Rückstellmittel
- 55: Drehfeder
- 56: Lagerstelle der Rotationstrommel
- 58: zweite Rotationsachse
- 60: geöffneter oberer Abschnitt des Schaftrohrs
- 62: Oberseite des Schaftrohrs
- 63: Unterseite des Schaftrohrs
- 64: Blickrichtung des Abbildungssystems
- 66: abgeflachte Seite der Rotationstrommel
- 68: Flexleiterplatte als Steuerleitung
- 69: erste Fluidleitung
- 70: Vertiefung auf der Rotationstrommel
- 71: Kabel
- 72: Werkzeug
- 74: Handgriff
- 76: Bedienelement
- 78: blasenförmiger Operationsbereich
- 79: Gewebe
- 80: Strömungsrichtung
- 82: Lenkungsglieder
- 84: äußerer Rand der Lenkungsglieder
- a: Abstand zw. der ersten Rotationsachse und der Aufwickelkurve
- d: Außendurchmesser des Schaftrohrs
- α: Winkel der Blickrichtung des Abbildungssystems
- β: Blickwinkel oder Blickfeld
- β': Beobachtungsbereich
- S: Verstellweg

## Patentansprüche

1. Endoskop (10) mit einer Rotationstrommel (20) oder einem Rotationsmodul, insbesondere für die Verwendung als ein steriles Einweginstrument, mit einem langerstreckten starren und/oder flexiblen Schaftrohr (12), das an einem distalen Ende (14) vorzugsweise mittels einer Lagergabel (16) die Rotationstrommel (20) um zumindest eine erste Rotationsachse (18) rotierbar lagert, in der ein optisches Abbildungssystem (22) angeordnet ist, wobei das optische Abbildungssystem (22) vorzugsweise einen elektronischen Bildaufnehmer (24) und/oder eine Abbildungsoptik (26) und/oder eine Beleuchtungseinrichtung (28) umfasst, wobei die zumindest eine erste Rotationsachse (18) etwa quer zu einer Längsachse (30) des Schaftrohrs (12) verläuft und wobei das Endoskop (10) zumindest eine Steuerleitung (32) aufweist, um die Rotationstrommel (20) vorzugsweise durch eine Linearbewegung zu rotieren,
**dadurch gekennzeichnet,**
**dass** die zumindest eine Steuerleitung (32) der Rotationstrommel (20) auf einer Außenseite/Außenfläche (36) des Schaftrohres (12), bevorzugt einen Arbeitskanal (34) im Inneren des Schaftrohres (12) und den Innendurchmesser des Schaftrohres (12) nicht einschränkend, verläuft und mit zumindest einem Hebelabstand (a) zu der zumindest einen ersten Rotationsachse (18) an der Rotationstrommel (20) befestigt ist.

2. Endoskop nach dem Anspruch 1,
**dadurch gekennzeichnet,**
**dass** die zumindest eine Steuerleitung (32) bevorzugt auf den äußeren Umfang der Rotationstrommel (20) entlang zumindest einer Aufwickelkurve (38) aufwickelbar ist.

3. Endoskop nach dem Anspruch 1 oder 2,
**dadurch gekennzeichnet,**
**dass** die zumindest eine Steuerleitung (32) der Rotationstrommel (20) als eine Versorgungsleitung für das Abbildungssystem (22), vorzugsweise als Flexleiterplatte (68) oder als ein Kabel (71), für elektronische Schaltkreise im Inneren der Rotationstrommel (20) ausgeführt ist.

4. Endoskop nach einem der Ansprüche 1 bis 3,
**dadurch gekennzeichnet,**
**dass** die zumindest eine Steuerleitung (32) in einer Aussparung (50), bevorzugt mit einem Schutzkragen (52), in der Außenfläche (36) des Schaftrohres (12) geführt ist, wobei die Aussparung (50) parallel zu der Längsachse (30) des Schaftrohres (12) verläuft.

5. Endoskop nach einem der Ansprüche 1 bis 4,
**dadurch gekennzeichnet,**
**dass** an der Rotationstrommel (20) Rückstellmittel (54) angeordnet sind, bevorzugt eine Drehfeder (55) in einer Lagerstelle (56) der Rotationstrommel (20).

6. Endoskop nach einem der Ansprüche 1 bis 5,
**dadurch gekennzeichnet,**
**dass** der Arbeitskanal (34) zumindest eine zweite Fluidleitung (39) umfasst, modular bevorzugt eine Luftleitung (39, 40) und/oder bevorzugt eine Flüssigkeitszufuhrleitung (39, 42) und eine Flüssigkeitsabfuhrleitung (39, 44) aufweist.

7. Endoskop nach dem Anspruch 6,
**dadurch gekennzeichnet,**
**dass** das Schaftrohr (12) an einem distalen Ende (14) umfangsseitig zumindest ein Loch (46), bevorzugt eine Vielzahl an Löchern (46), aufweist, das die zumindest eine zweite Fluidleitung (39), bevorzugt die Flüssigkeitsabfuhrleitung (39, 44), mit einem Operationsbereich verbindet, um ein Spülfluid aus einem Operationsbereich zu entfernen.

8. Endoskop nach einem der Ansprüche 1 bis 7,
**dadurch gekennzeichnet,**
**dass** der Arbeitskanal (34) zur Aufnahme zumindest eines Instruments (48) ausgebildet ist und/oder zumindest ein Instrument (48) aufweist.

9. Endoskop nach dem Anspruch 8,
**dadurch gekennzeichnet,**
**dass** das Schaftrohr (12) an einem oberen Abschnitt (60) geöffnet ist, wobei die zumindest eine Steuerleitung (32) an einem unteren Abschnitt (61) geführt ist, sodass vorzugsweise zumindest ein flexibles Instrument (48) in eine Richtung senkrecht zu der Längsachse (30) des Schaftrohrs (12) verstellbar/biegbar ist.

10. Endoskop nach einem der Ansprüche 1 bis 9,
**dadurch gekennzeichnet,**
**dass** die Lagergabel (16) schwenkbar um eine zweite Rotationsachse (58) an dem Schaftrohr (12) befestigt ist und der Arbeitskanal (34) in einem geschwenkten Zustand der Lagergabel (16) entlang der Längsachse (30) geöffnet ist, um vorzugsweise das zumindest eine Instrument (48) aus dem Arbeitskanal (34) in einen Operationsbereich zu führen, wobei die Lagergabel (16) vorzugsweise mit einer weiteren Steuerleitung steuerbar ist.

11. Endoskop nach einem der Ansprüche 1 bis 10,
**dadurch gekennzeichnet,**
**dass** der Außendurchmesser (d) des Schaftrohres (12) 3mm bis 6mm beträgt.

12. Endoskop nach einem der Ansprüche 2 bis 11,
**dadurch gekennzeichnet,**
**dass** mehrere Steuerleitungen (32) auf unterschiedlichen Aufwickelkurven (38) entlang des Umfangs der Rotationstrommel (20) aufwickelbar sind, insbesondere mit einem unterschiedlichen Abstand (a) zu der Rotationsachse (30), um bei gleichem Verstellweg (S) der zumindest einen Steuerleitung (32) eine unterschiedliche Drehverstellung der Rotationstrommel (20) auszuführen.

13. Verfahren zum Bedienen eines Endoskops (10), insbesondere nach einem der Ansprüche 1 bis 12, um eine Rotationstrommel (20) mit einem Abbildungssystem (22), vorzugsweise mit einem elektronischen Bildaufnehmer (24) und/oder einer Abbildungsoptik (26) und/oder einer Beleuchtungseinrichtung (28), zu steuern, wobei zumindest eine Steuerleitung (32) auf einer Außenseite/Außenfläche (36) eines Schaftrohres (12) des Endoskops (10) und einen Arbeitskanal (34) im Inneren des Schaftrohres (12) nicht einschränkend angeordnet und mit zumindest einem Hebelabstand (a) zu einer zumindest einen ersten Rotationsachse (18) an der Rotationstrommel (20) befestigt wird und wobei anschließend an der zumindest einen Steuerleitung (32) gezogen oder gedrückt wird, um die Rotationstrommel (20) zu rotieren.

14. Verfahren nach dem Anspruch 13 mit einem Endoskop (10),
insbesondere nach einem der Ansprüche 7 bis 13, um ein Blickfeld (β) des Abbildungssystems (22) zu reinigen mit den folgenden Schritten:
- Rotation der Rotationstrommel (20) von einer Beobachtungsstellung eines Operationsbereichs in eine Reinigungsstellung, in der das Blickfeld (β) auf das distale Ende (16) des Schaftrohrs (12) und den Arbeitskanal (34) ausgerichtet ist.
- Spülung des Abbildungssystems (22) und/oder der Abbildungsoptik (26) und/oder der Beleuchtungseinrichtung (28) und insbesondere in dem Blickfeld (β) mit einem Spülfluid aus zumindest einer zweiten Fluidleitung (39).
- Entfernen des Spülfluids mit einer Fluidströmung, bevorzugt einer Luftstörmung, aus einer weiteren zweiten Fluidleitung (39), insbesondere einer Luftleitung (39, 40).
- Rotation der Rotationstrommel (20) zurück in die Beobachtungsstellung.

15. Verfahren nach dem Anspruch 13 oder 14 mit einem Endoskop (10), insbesondere nach den Ansprüchen 9, 10 und 11 mit den folgenden Schritten:
- Führen des Schaftrohres (12), zumindest mit dem distalen Ende (14), in einen Operationsbereich.
- Schwenken einer Lagergabel (16) um eine zweite Rotationsachse (58), zumindest bis die Rotationstrommel (20) den Arbeitskanal (34) entlang einer Längsachse (30) des Schaftrohres (12) freigibt.
- Führen des zumindest einen Instruments (48) aus dem Arbeitskanal (34) heraus, insbesondere in den medizinischen Operationsbereich hinein.
- Schwenken der Rotationstrommel (20) mittels der Steuerleitung (32) um eine erste Rotationsachse (18), um das zumindest eine Instrument (48) mit einem Blickfeld (β) des Abbildungssystems (22) im Operationsbereich zu verfolgen.
- Schwenken der Lagergabel (16) um eine zweite Rotationsachse (58), um das zumindest eine Instrument (48) zu bewegen/biegen.
